# EUROPEAN PATENT APPLICATION

(11) **EP 1 437 042 A1**
(43) Date of publication of application: **14.07.2004**
(21) Application number: 02767910.9
(22) Date of filing: 06.09.2002
(51) Int. Cl.: A01K 67/027, C12N 5/06

(54) **HAIR FOLLICLE-RECONSTITUTION SYSTEM AND ANIMAL CARRYING THE SAME**

(30) Priority: 06.09.2001 JP 2001270100
(71) Applicant: SHISEIDO COMPANY, LTD., Tokyo 104-8010 (JP)
(72) Inventor: IDETA, Ritsuro, c/o Shiseido Research Center, Yokohama-shi, Kanagawa 224-8558 (JP); TSUNENAGA, Makoto, c/o Shiseido Research Center, Yokohama-shi, Kanagawa 224-8558 (JP)
(74) Representative: Albrecht, Thomas, Dr.
(86) International application number: PCT/JP2002/009082
(87) International publication number: WO 2003/022043

(57) **Abstract**

This invention provides an experimental animal which carries hair follicles which have been reconstituted with use of hair papilla cells, epidermal cells and melanocytes which are extraneous to these cells. Such an experimental animal is usable for evaluating factors which influence hair restoration and the depth of hair color.

## Description

### Technical Field

This invention relates to experimental animals which carry a hair-follicle reconstitution system, and to a cell-system by which to prepare said experimental animals and also to the use thereof.

### Backaround Art

Various kinds of chimera animals and transgenic animals which can stably reproduce animal reaction that is directed to a specific objective have been developed chiefly as a model of specific diseases or for the purpose of explicating the cause of specific diseases. For example, there has been developed a nude mouse, to be used for assaying hair-follicle reconstitution, on which hair germ (hair-follicle progenitor cells which exist as keratinocyte agglomeration on the skin of neonatal mouse) has been transplanted together with hair-inducible dermal papilla cells (derived from mustache of rat) (Lichti et al., J. Invest. Dermatol. 101: 124129, 1993).

Furthermore, in a mouse on which both cultured keratinocytes derived from hair germ of neonatal mouse and dermal papilla cells have been transplanted, it is confirmed by means of histological analysis of transplantation region that epithelium and hair follicles have been completely reconstituted as tissues (Kamimura et al., J. Invest. Dermatol. 109: 534540, 1997). It has been suggested that such reconstituted hair follicles are usable for the reliable functional assay of *in vivo* hair-induction.

A phenomenon which is to be referred to together with hair induction is the color tone of hair. The color tone of hair is considered, in most cases, to be given by melanin which is produced by melanocytes in the vicinity of hair matrix and is supplied to hair-forming cells. It is said that the main cause of the graying of hair is that such melanocytes have ceased to produce melanin or produce only a very small amount of melanin, or that melanocytes have decreased or disappeared. In connection with hair cycle, however, it is unknown how melanocytes, which have sharply decreased or disappeared from hair follicles through catagen (a period when hair ceases to grow, hair follicles are shortened, and club hair is formed) and telogen, is complemented in the next anagen and adjusts the color tone of hair (or, it is unknown from where melanocytes come), etc.

Thus, it would be worthwhile to provide a means to adjust the color tone of hair, or an *in vivo* evaluation system which can evaluate whether or not a certain means is capable of preventing the graying of hair or has an anti-graying effect. Hence, the objective of the present invention is to provide such an evaluation system. In this specification, "anti-graying activity" or "anti-graying effect" means a hair graying-inhibitory property which can be evaluated by at least one activity of melanocytes selected from the group consisting of growth acceleration, migration activity acceleration, differentiation ability acceleration, survivability improvement and melanin-productivity, which activity is indicated by the color of hair.

### Disclosure of Invention

In their study for the purpose of attaining the above-mentioned objective, the inventors of this invention have found out that so-called reconstituted hair follicles as explained above can be obtained by the addition of extraneous melanocytes whether they may be homogenous or heterogenous, and that melanocytes in thus reconsitituted hair follicles can be maintained active.

Thus, this invention provides a system for hair follicle-reconstitution which comprises a combination of dermal papilla cells and epidermal cells and further melanocytes which are extraneous to these cells.

As another embodiment, this invention provides a reconstituted hair follicles-carrying animal which is prepared by the transplantation of the above-mentioned system onto a recipient animal.

As another embodiment, this invention further provides a method to evaluate the melanocyte-activating capability of a certain means which method is characterized by preparing the above-mentioned chimera animal as a subject, treating said subject animal with a certain means, monitoring the activity of melanocytes in reconstituted hair follicles of thus treated subject animal, and finding a correlation between the degree of change of said activity in comparison with an untreated control and the melanocyte-activating capability of said means.

### Brief Description of Drawings

Figure 1 is a photograph in place of a drawing which shows the state of back portion of chimera mouse which has been prepared in Example II-2. Numerals in this Figure are experimental numbers.
Figure 2 is a graph which shows the changes in melanin content in restored hair of chimera mouse of the present invention under the action of the several kinds of factors in accordance with Example III.

### Best Mode for Carrying Out the Invention

In the present specification, the term "dermal papilla cells" (hereinafter sometimes referred to as DP) means a concept which broadly includes cells which constitute a tissue called dermal papilla in the lowermost part of hair follicle, and dermal papilla with its peripheral cells and tissues so long as they serve to achieve the objective of this invention. An example of such a tissue is dermis which contains hair papilla cells. Although non-restrictive, such cells, when mouse-derived ones are taken as an example, can be obtained from a neonate (to be used within four days of birth) which is born from a transgenic mouse (Ver-LacZ) into which an expression vector composed of a suitable reporter gene [e.g., LacZ gene, green fluorescent protein (GFP)] connected to the downstream of Vercican promoter has been introduced, by taking the expression of the reporter gene as a sign. As for dermis which contains hair papilla cells, dermis which is taken from skin by a normal preparation method usually contains hair papilla cells, and is therefore also usable, as it is, for dermal papilla cells of this invention.

"Epidermal cells", on the other hand, are cells which constitute the major part of epidermis or epithelium of skin, and are formed from a layer of basement cells which is in contact with dermis. When mouse is taken as an example, epidermal cells derived from neonate (or fetus) are preferably used as the above-mentioned epidermal cells. Also usable is cell culture in the form of keratinocyte. Such cells may be prepared from skin of desired donor animal by any known method.

The above-mentioned hair papilla cells or dermis which contains hair papilla cells and epidermal cells are usable regardless of the species of donor animal so long as they can be transplanted on recipient animal. Donor animal is, however, preferably homogenous to recipient animal. Although non-restrictive, when the recipient animal is mouse, both of the above-mentioned cells may be derived from mouse. On the other hand, both of the cells do not need to be derived from the same strain of mice. It is therefore acceptable that hair papilla cells or dermis which contains hair papilla cells are obtained from either of the above-mentioned transgenic mouse (e.g., Ver-LacZ strain) in which hair papilla cells are easily confirmed, and that epidermal cells are obtained from mouse selected from the group consisting of ICR strain having albinic nature (with a genetic defect in tyrosinase) and melanocyte-deficient strain (e.g., W^{sh}/W^{sb} mouse). According to this invention, epidermal cells obtained from such an albinic or melanocyte-deficient animal are preferably used since they facilitate the monitoring of the behavior of extraneous melanocytes as mentioned later. Also the above-mentioned transgenic mouse may be derived from mouse selected from the group consisting of ICR strain and melanocyte-deficient strain.

In this specification, the term "extraneous" in "extraneous melanocytes" means that melanocytes are different in origin from hair papilla cells and epidermal cells. Hence, melanocytes include not only melanocytes derived from an animal of a species different from that of animals from which hair papilla cells or epidermal cells are derived, but also melanocytes derived from an animal which is the same in terms of species and strain as, but is individually different from, animals from which hair papilla cells or epidermal cells are derived. This means that, even though endogenous melanocytes are mixed with a preparation of hair papilla cells or epidermal cells, additional melanocytes other than said endogenous ones are necessarily contained in the intended system.

Although non-restrictive, however, when hair papilla cells and epidermal cells are derived from mouse, melanocytes are preferably derived from an animal other than mouse, e.g., human being. Such a preferable combination makes it possible to trace, in reconstituted hair follicles, the distribution of melanocytes regardless of the state of differentiation of melanocytes (e.g., the use of antibody to human melanocytes or of specific antibody to human being, or the use of specific gene sequence of human being or of specific gene sequence of human melanocytes). This invention is characterized also in that, in so-called chimera reconstituted hair follicles which comprise hair papilla cells (mouse), epidermal cells (mouse) and melanocytes (human being), melanocytes maintain their activity (e.g., melanin-producing activity). This invention is further characterized by its capability of searching out means which can act on human melanocytes in an evaluation method as mentioned later.

Melanocytes to be used in this invention may be derived from animal of any species so long as they can produce melanin to achieve the objective of this invention. In order to make the best use of the above-mentioned characteristic features, however, human-derived melanocytes are preferably used as explained above. Melanocytes may be prepared from any suitable tissue such as epidermis and foreskin by any publicly known method. Products on the market are also usable. For example, human-derived cultured melanocytes are available from Cascade Co. or Kurashiki Boseki K.K. under the name of NHEM cells.

Hair papilla cells and epidermal cells, on the other hand, are preferably derived from animal of the same species as recipient animal. Concrete examples of such an animal include mouse and rat in view of the objective of this invention.

The "system for reconstituting hair follicles" of this invention comprises a combination of the above-mentioned hair papilla cells, epidermal cells and extraneous melanocytes. The wording "system ... comprises ..." not only means that the system includes the above-mentioned cells as one, but also means an embodiment wherein the cells are separately stored, e.g., kept in separate containers, so that they may be used as one in the future. Hence, so long as the cells are used for the purpose of achieving the above-mentioned system, it falls within the scope of this invention. The phrase "for reconstituting hair follicles" means that the system can bring about reconstituted hair follicles (or chimeric hair follicles) which serve as an organ to support hair restoration or hair growth in recipient animal. Examples of such an organ include hair papilla cells, hair matrix, follicular epidermal cells and tissues (e.g., root sheath), sebaceous glands and connective tissue which surrounds hair follicles.

When transplanted onto a suitable recipient animal, the system of this invention as mentioned above provides a chimera animal which carries reconstituted hair follicles. Such a chimera animal has active (preferably xenogenic animal-derived) melanocytes in hair follicles, and is therefore useful for the evaluation of means (including drug and environment) which may influence the function or activity of melanocytes. Recipient animal is preferably an immunodeficient animal regardless of the origin of cells which are contained in the system to be transplanted on said animal. As for the species of such an animal, any will do so long as the animal of the species is usable as an experimental animal and meets the purpose of this invention. Preferable examples include mouse and rat. Immunodeficient ones among these animals include nude mouse which has a thymus deficient phenotype if mouse is to be taken as an example. When the purpose of this invention is taken into consideration, nude mouse (e.g., Balb/c nu/nu strain), SCID mouse (e.g., Balb/c SCID) and nude rat (e.g., F344/N Jcl rnu), each on the market, are in particular desirable as recipient animal. Reconstituted hair follicles which are obtained by using such a recipient animal as mentioned above, and by using such epidermal cells as derived from albinic mouse (e.g., ICR strain) or melanocyte-deficient mouse (e.g., W^{sh}/W^{sh}) are especially preferable since it is easy to trace the activity of melanocytes which are contained in said hair follicles.

According to this invention, even though the above-mentioned melanocytes are derived from human being, melanin-containing hair is induced and grown from reconstituted hair follicles so long as the above-mentioned especially preferable recipient animal and epidermal cells are used. The production of melanin is observed also in a region which surrounds hair follicles. Furthermore, according to the observation of hair by stereoscopic microscope six weeks after the transplantation, the color of hair does not change, e.g., from gray to white, during said period, and, thus, it is confirmed that melanin-producing melanocytes exist in reconstituted hair follicles for a long time. Moreover, when a large amount of human-derived melanocytes is mixed in transplantation, the color of hair generally looks dark. On this account, it is considered that the color of reconstituted hair depends on the amount of melanin produced by human-derived melanocytes around dermal papilla cells.

The above-mentioned system of this invention for reconstituting hair follicles may be transplanted on recipient animal by any publicly known method. For instance, when said system is to be transplanted on the back portion of nude mouse, there are used about 500,000 to 10,000,000, preferably about 1,000,000 to about 4,000,000, dermal papilla cells, about 1,000,000 to 40,000,000, preferably about 10,000,000 to about 20,000,000, mouse epidermal cells, and about 500,000 to 10,000,000, preferably about 1,000,000 to 5,000,000, cultured human melanocytes (DARK) in a circle with a diameter of 1 cm on said back portion.

In a chimera animal which carries such reconstituted hair follicles as mentioned above, melanin-containing hair is induced from reconstituted hair follicles and grown for a long period of time. It is considered therefore that cells, tissue or organ such as melanocytes, hair papilla and hair matrix which participate in hair-growing or hair color thickening are working normally. Thus, chimera animal in accordance with this invention is usable for:
1) the screening of drugs which have an action of activating melanocytes and thereby deepening the hair color;
2) the screening of drugs which have an action of activating hair papilla cells and thereby accelerating the hair growth and deepening the color of whole hair;
3) the screening of drugs which have an action of stimulating hair matrix and thereby accelerating the hair growth and deepening the color of whole hair;
4) the screening of drugs which have an action of stimulating hair papilla cells and thereby activating melanocytes in hair follicles and deepening the color of whole hair;
5) the screening of drugs which have an action of stimulating hair matrix and thereby activating melanocytes in hair follicles and deepening the color of whole hair; and
6) drugs which deepen the hair color by some of the above-mentioned actions or a combination of all of the actions, and the screening of drugs having said combined action.

Incidentally, melanocyte-activating drugs or melanocyte-activating chemicals in accordance with this invention include drugs which deepen the hair color by stimulating and activating some or all of the growth, differentiation, proliferation, survival and migration activity of melanocytes, the activity being indicated by hair color.

An embodiment of this invention which is to be used in the above-explained manner is a method to evaluate melanocyte-activating ability which comprises:
(A) a step of preparing a chimera animal as a subject animal;
(B) a step of treating said subject animal by a certain means, and monitoring the activity of melanocytes in reconstituted hair follicles of thus treated subject animal; and
(C) a step of finding the correlation between the degree of change of activity in the step (B) in comparison with untreated control [e.g., the activity of melanocytes in reconstituted hair follicles of subject animal which has not undergone the treatment of step (B)] and the melanocyte-activating ability of said means.

In the above-mentioned method, melanocyte-activating ability can be evaluated by the amount of melanin in hair which has been regrown from reconstituted hair follicles. It may also be possible to evaluate, by the degree of hair restoration and hair growth, the influence of the means of step (B) on one or both of dermal papilla cells and hair matrix. Examples of such a means include environment in which the subject animal is put, e.g., a stressful environment and a relaxing environment in contrast thereto, drugs which are applied to reconstituted hair follicles of subject animal or which are injected subcutaneously to subject animal, or drugs with which melanocytes are previously treated, or drugs which are perorally administered. Drugs may be added to the above-mentioned system for reconstituting hair follicles when a reconstituted hair follicles-carrying chimera animal is prepared.

The above-mentioned monitoring of melanocytes may be conducted by assaying, through any known method, enzyme *per se* in melanin-biosynthesis course or by assaying DNA or mRNA which encodes such an enzyme, when reconstituted hair follicles are to be prepared with use of human-derived melanocytes. In another method, there may be measured melanin content in a hair which has newly been regrown or grown after a hair of chimera animal is pulled out or cut, or in a hair which has newly been regrown after one hair cycle is over.

This invention is further concretely explained by the following examples, which are provided for the purpose of facilitating the understanding of this invention, and are not intended to restrict the scope of this invention.

### I. Examples of preparation of cells

### Example 1: Mouse-derived hair papilla cells

(1-1) There is selected a LacZ positive one from neonates (to be used within four days of birth) which are born from transgenic mouse into which there has been introduced an expression vector which is prepared by connecting structural gene of a suitable marker protein (e.g., LacZ) to the downstream of Vercican promoter.
(1-2) Each mouse is washed with ethanol and suitable washing liquid (e.g., phosphate-buffered saline; to be abbreviated to PBS). Then, skin on the back portion is peeled off, and is left still overnight in 0.25 % trypsin/PBS at 4°C.
(1-3) Next day, epidermis and dermis are separated from each other by use of pincette or the like. Then, the side which contains dermis is treated with 0.35 % collagenase/DMEM (Dulbecco-Modified Eagle's Minimum Essential Medium) for about one hour at 37°C.
(1-4) The sample of (1-3) is subjected to a careful suspension operation, and is then passed through a cell strainer having a pore size of 70 µm. Thus treated sample is subsequently centrifuged, and, thus, cells are collected.
(1-5) Thus collected cells are subjected to a suitable cell sorter, and so, only cells in which LacZ gene has been expressed are recovered. Thus recovered cells are cultivated in a suitable culture liquid [e.g., DMEM + 10 % fetal bovine serum (to be abbreviated to FBS)], or are freeze-preserved in a normal cell-freezing solution up to the day before use.
(1-6) The cells are placed under a suitable culture condition (e.g., DMEM + 10 % FBS in 5 % CO₂ at 37°C) up to the day before use, and, on the next day, are adjusted with use of trypsin or the like immediately before the operation.

### Example 2: Mouse-derived epidermal cells

(2-1) On the day before operation, skin from neonate of albinic mouse (e.g., ICR strain) is treated with trypsin by the same manner as in (1-1) and (1-2).
(2-2) Only epidermal portion is peeled off with use of pincette or the like. Said epidermal portion is cut fine, and is then subjected to a suspension treatment in a suitable culture liquid (e.g., keratinocyte culture medium, to be abbreviated to KGM) at 4°C for about one hour.
(2-3) The sample of (2-2) is passed through a cell strainer having a pore size of 70 µm, and is subsequently centrifuged, and, thus, epidermal cells are recovered.
(2-4) Per one recipient animal, there are used, for operation, epidermal cells in an amount corresponding to two neonates. Cells in said amount are suspended with use of KGM, and are then left to stand still on ice until immediately before use. Or, otherwise, the recovered cells may be freeze preserved, to be defrosted before use.

### Example 3: Mouse-derived dermal cells

(3-1) On the day before operation, skin from neonate of albinic mouse (e.g., ICR strain) is treated with trypsin by the same manner as in (1-1) and (1-2).
(3-2) Epidermal portion is peeled off with use of pincette or the like. Remaining dermis is cut fine, and is then subjected to a suspension treatment in a suitable culture liquid (e.g., DMEM + 10 % FBS) which contains 0.35 % collagenase at 37°C for about one hour.
(3-3) The sample of (3-2) is passed through a cell strainer having a pore size of 100 µm, and is subsequently centrifuged, and, thus, dermal cells are recovered.

Per one recipient animal, there are used, for operation, dermal cells in an amount corresponding to two neonates. These dermal cells are not used simultaneously with isolated hair papilla cells. Cells in said amount are suspended with use of a liquid such as DMEM + 10 % FBS, and are then left to stand still on ice until immediately before use or freeze-preserved.

### Example 4: Human-derived cultivated melanocytes

(4-1) Foreskin-derived melanocytes on the market (e.g., NHEM cells sold by Cascade Co.) are cultivated in a culture liquid for melanocytes (e.g., M154s medium of Cascade Co.). The melanocytes are made to proliferate up to an amount corresponding to 500,000 to 10,000,000 cells per one recipient animal, by the day of operation.
(4-2) Thus proliferated melanocytes are, immediately before used, peeled off the incubator by means of a treatment with 0.05 % trypsin, and are then suspended in a culture liquid, and are subsequently left to stand still on ice until immediately before used.

### Example 5: Mouse-derived melanocytes

(5-1) Skin is isolated, by a method in accordance with (1-1) and (1-2), from neonatal mouse (C57Black/6 strain is used for this Example) more than one week before operation, and is then treated with trypsin.
(5-2) Epidermis is peeled off, and is then floated on PBS which contains 0.02 % EDTA.
(5-3) After gentle mixing, the epidermis floated on PBS is shaken at 37°C for about eight minutes.
(5-4) Thus treated sample is passed through a cell strainer having a pore size of 70 µm, and is subsequently centrifuged, and, thus, cells are recovered, which are then cultivated in a culture liquid for melanocytes. Cultivating conditions depend on the state of cells.
(5-5) Thus cultivated melanocytes are, immediately before used, peeled off the incubator by means of a treatment with 0.05 % trypsin, and are then suspended in a culture liquid, and are subsequently left to stand still on ice until immediately before used, or freeze-preserved.

### II. Method to reconstitute hair follicles (method of transplantation onto animal)

### Example II-1: A case where mouse-derived hair papilla cells are used:

Necessities: The following II-(1-1), (1-2) and (1-3), each in a suitable amount, are mixed to be used as "cell suspension liquid" for "process to prepare reconstituted hair follicles".
II-(1-1) Dermal papilla cells (hereinafter referred to as DP) which are prepared from dermis of neonates born from transgenic mouse into which there has been introduced an expression vector which is prepared by connecting structural gene of a suitable marker protein (e.g., LacZ) to the downstream of Vercican promoter.
II-(1-2) Mouse-derived epidermal cells. On the day before operation, skin is taken from neonate of albinic mouse (e.g., ICR strain), and, on the day of operation, is treated with trypsin. Thus prepared cells may be used after freeze-preserved.
II-(1-3) Cultivated melanocytes (A or B is used):
   A. Human-derived melanocytes. Foreskin-derived melanocytes on the market are subjected to subculture (e.g., NHEM cell sold by Cascade Co., etc.). Prepared by a treatment with trypsin on the day of operation. Thus prepared cells may be used after freeze-preserved.
   B. Mouse-derived melanocytes. Isolated from neonatal mouse (C57Black/6 strain is used for this Example) more than one week before operation, and are then moved to culture system for subculture. Prepared by a treatment with trypsin on the day of operation. Thus prepared cells may be used after freeze-preserved.

### Example II-2: A case where mouse dermis is used:

In place of the above-mentioned II-(1-1), skin is taken, on the day before operation, from neonate of albinic mouse (e.g., ICR strain), and, on the day of operation, is treated with collagenase, and, thus, "cell suspension liquid" is prepared. Thus prepared cell suspension liquid may be used after freeze-preserved.

### <<Process to Prepare Reconstituted Hair Follicles>>

### Necessities:

Recipient animal (e.g., Balb/c nu/nu strain nude mouse; at least five-week old);

Silicone-made dome-like cap having a diameter of about 1 cm (hereinafter referred to as valve);

Anesthetic;

Operating scissors, pincette, suturing apparatus;

Micropipette;

"Cell suspension liquid": Comprises melanocytes, epidermal cells, dermal cells or dermal papilla cells. Cells are each prepared in the above•mentioned manner. Cells are suspended in about 150 µl of culture liquid (such as DMEM + 10 % FBS), and are then left to stand still on ice or freeze-preserved, from which cell suspension liquid is prepared immediately before operation.

### <<Process>>

(i) Nude mouse is anesthetized.
(ii) A piece of skin in a diameter of a little less than 1 cm is cut out of back portion.
(iii) Valve is inserted into wound, and is fixed with a suturing apparatus.
(iv) Cell suspension liquid is injected into valve with use of pipette.
(v) The mouse is kept in this state for about one week, and, then, the valve is removed.
(vi) After one or two weeks, reconstituted hair follicles are seen to grow in the place from which a scaff has fallen off. Thus, it can be observed that, when melanocytes are added to the suspension liquid, the hair, which should otherwise be albinic pure white, has turned gray.
(vii) Histological observation definitely teaches that this hair color is caused by melanin.

### Results

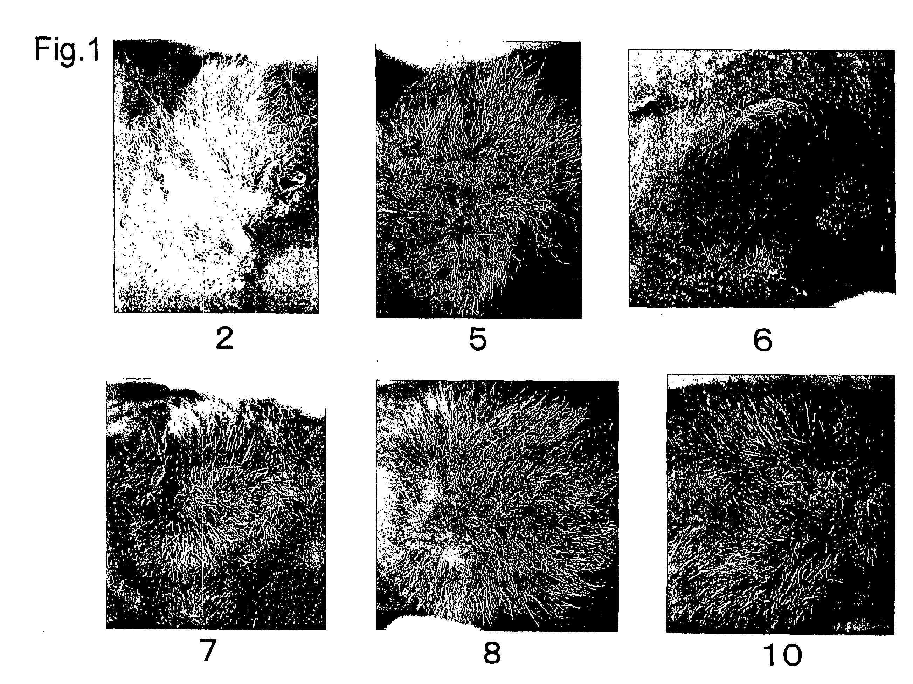

The following Table 1 shows concrete conditions (system for reconstituting hair follicles; mark + indicates cells which are contained in system) for preparing reconstituted hair follicles-carrying animal in accordance with the above-mentioned cells and process, hair color and the skin color of the portion of transplantation. Attached Figure 1 is a photograph in place of drawing which shows the state of back portion of animals prepared by Experiment Nos. 2, 5, 6, 7, 8 and 10.

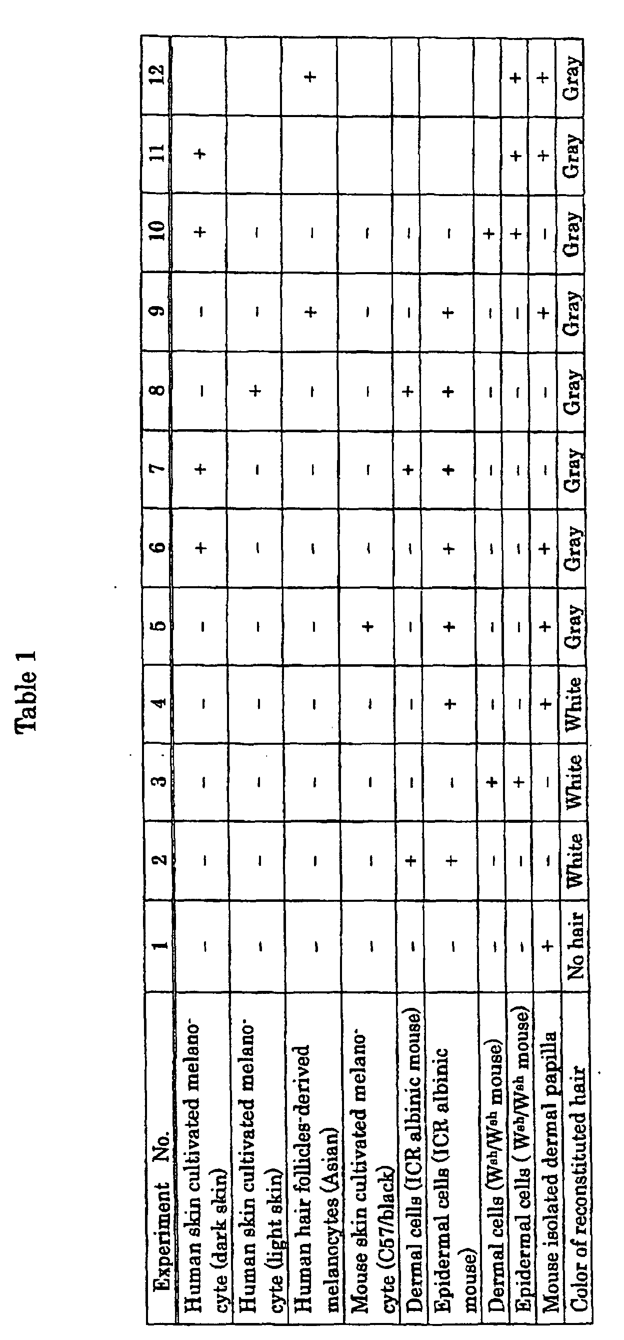

### Example III Evaluation of anti-hair graying drugs

In accordance with «Process» of Example II-2, the system of Experiment No. 7 for the reconstitution of hair follicles was transplanted onto nude mice. After about three to four weeks, reconstituted hair was pulled out. After the hair was pulled out, solutions which respectively contained stem cells factor (hereinafter referred to as SCF; 30 ng per gram of animal), α-melanocytes-stimulating hormone (hereinafter referred to as MSH; 1 pmol per gram of animal) and *p*-aminobenzoic acid (hereinafter referred to as PABA; 50 ng per gram of animal) or phosphate-buffered saline (PBS: for control) were subcutaneously injected to mice every day, which was repeated for one week.

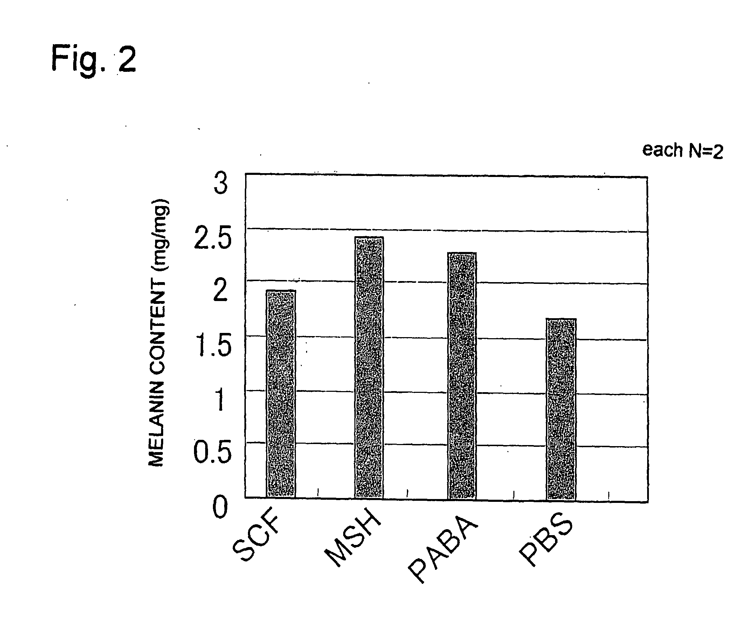

After the hair which had been restored again grew sufficiently (about after three weeks), thus restored hair was cut with scissors and collected. After the hair was dissolved, melanin content was measured by absorbance. Results are shown in Fig. 2. Fig. 2 shows that the addition of each factor significantly increases melanin content in restored hair. Thus, the chimera animal of this invention is usable at least for the screening of anti-hair graying drugs.

### Industrial Applicability

This invention provides chimera animal with a stable phenotype which is suitable for the screening of anti-hair graying drugs. This invention is therefore applicable in experimental animal-supplying industries, and also in medical industries, cosmetic industries, etc., which develop effective anti-hair graying drugs with use of said animals.

## Claims

1. A system for hair follicle-reconstitution which comprises a combination of hair papilla cells or hair papilla cells-containing dermal cells, epidermal cells and melanocytes which are extraneous to these cells.

2. A system of claim 1 wherein extraneous melanocytes are of origin which is extraneous to hair papilla cells, hair papilla cells-containing dermal cells and epidermal cells.

3. A system of claim 1 wherein extraneous melanocytes are derived from human being, wherein hair papilla cells or hair papilla cells-containing dermal cells and epidermal cells are derived from the same or different homogenous animal strain, and wherein said animal is selected from the group consisting of mouse and rat.

4. A system of claim 1 wherein extraneous melanocytes are derived from human being, wherein hair papilla cells-containing dermal cells are derived from mouse selected from the group consisting of ICR-strain albinic mouse and melanocyte-deficient strain mouse, and wherein epidermal cells are derived from mouse selected from the group consisting of ICR-strain albinic mouse and melanocyte-deficient strain mouse.

5. A system of claim 1 wherein extraneous melanocytes are human skin cultivated melanocytes, wherein hair papilla cells or hair papilla cells-containing dermal cells are selected from the group consisting of mouse-isolated hair papilla cells, ICR-strain albinic mouse-derived dermal cells and W^{sh}/W^{sh} mouse-derived dermal cells, and wherein epidermal cells are selected from the group consisting of ICR-albinic mouse-derived epidermal cells and W^{sh}/W^{sh} mouse-derived epidermal cells.

6. A reconstituted hair follicles-carrying chimera animal which is prepared by the transplantation, onto a recipient animal, of a system for hair follicle-reconstitution which comprises a combination of hair papilla cells or hair papilla cells-containing dermal cells, epidermal cells and melanocytes which are extraneous to these cells.

7. A chimera animal of claim 6 wherein recipient animal is an immunodeficient animal and extraneous melanocytes are derived from human being.

8. A chimera animal of claim 6 wherein recipient animal is an immunodeficient animal which is selected from the group consisting of nude mouse, SCID mouse and nude rat.

9. A chimera animal of claim 6 wherein recipient animal is nude mouse, extraneous melanocytes are derived from human being, hair papilla cells-containing dermal cells are derived from mouse selected from the group consisting of ICR-strain albinic mouse and melanocyte-deficient strain mouse, and wherein epidermal cells are derived from mouse selected from the group consisting of ICR-strain albinic mouse and melanocyte-deficient strain mouse.

10. A chimera animal of claim 6 wherein recipient animal is nude mouse, extraneous melanocytes are derived from human skin cultivated melanocytes, hair papilla cells or hair papilla cells-containing dermal cells are selected from the group consisting of mouse-isolated hair papilla cells, ICR-albinic mouse-derived dermal cells and W^{sh}/W^{sh} mouse-derived dermal cells, and wherein epidermal cells are selected from the group consisting of ICR-albinic mouse-derived epidermal cells and W^{sh}/W^{sh} mouse-derived epidermal cells.

11. A method to evaluate the melanocyte-activating capability of a certain means which method is **characterized by**:
preparing, as a subject, a reconstituted hair follicles-carrying chimera animal by means of transplanting, onto a recipient animal, a system for hair follicle-reconstitution which comprises a combination of hair papilla cells or hair papilla cells-containing dermal cells, epidermal cells and melanocytes which are extraneous to these cells;
treating said subject animal with said certain means;
monitoring the activity of melanocytes in reconstituted hair follicles of thus treated subject animal; and
finding a correlation between the degree of change (or difference) of said activity in comparison with an untreated control and the melanocyte-activating capability of said means.

12. An evaluation method of claim 11 wherein melanocytes are derived from human being, and the degree of change in the activity of melanocytes is determined by the amount of melanin either in hair which has been regrown from reconstituted hair follicles or in epidermis near said hair follicles.

13. An evaluation method of claim 11 wherein the melanocyte-activating capability is detected for the sake of the evaluation of anti-hair graying activity.

14. An evaluation method of claim 11 wherein said means is a chemical substance or a drug.

15. An anti-hair graying agent which comprises, as an effective ingredient, a drug which has been found, by the evaluation method of claim 11, to be capable of enhancing the activity of melanocytes.
